## Europäisches Patentamt
## European Patent Office
## Office européen des brevets

(11) Veröffentlichungsnummer: **0 111 209**
**B1**

(12) **EUROPÄISCHE PATENTSCHRIFT**

(45) Veröffentlichungstag der Patentschrift:
08.11.89

(51) Int. Cl.⁴: **C 07 C 31/26**, C 07 C 29/14, A 61 K 47/00 // A61K9/20

(21) Anmeldenummer: 83111682.7

(22) Anmeldetag: 23.11.83

(54) Verbesserter Sorbit, Verfahren zur Herstellung und Verwendung.

(30) Priorität: 07.12.82 DE 3245170

(43) Veröffentlichungstag der Anmeldung:
20.06.84 Patentblatt 84/25

(45) Bekanntmachung des Hinweises auf die Patenterteilung:
08.11.89 Patentblatt 89/45

(84) Benannte Vertragsstaaten:
AT CH DE FR GB IT LI NL

(73) Patentinhaber: MERCK PATENT GESELLSCHAFT MIT BESCHRÄNKTER HAFTUNG, Frankfurter Strasse 250 Postfach 4119, D-6100 Darmstadt (DE)

(72) Erfinder: Reiff, Friedrich, Dr., Im Rassdorf 6, D-6104 Seeheim 1 (DE)
Erfinder: Härtner, Hartmut, Dr., Mathildenweg 9, D-6109 Mühltal (DE)
Erfinder: Basedow, Arno, Dr., Elisabethenstrasse 25, D-6328 Bad Vilbel (DE)
Erfinder: Hugenbusch, Hans-Wolfgang, Hochstrasse 55, D-6128 Höchst 1 (DE)
Erfinder: Schmidt, Peter C., Prof. Dr., Am Hasenküppel 31, D-3550 Marburg (DE)
Erfinder: Bardonner, Hans, Erbacher Strasse 12, D-6123 Bad König (DE)

(56) Entgegenhaltungen:
EP-A- 0 032 288
DD-A- 35 860
DE-A- 1 941 956
DE-A- 2 059 246
DK-A- 133 603
FR-A- 1 040 994
FR-A- 2 451 357

CHEMICAL ABSTRACTS, Band 100, Nr. 2, 9. Januar 1984, Zusammenfassung Nr. 12550a, Columbus, Ohio (US); P.C. SCHMIDT: "Tableting characteristics of sorbitol."

(56) Entgegenhaltungen: (Fortsetzung)
Sorbit In der Medizin und Pharmazie, Deutsche Maizena Weke GmbH, 2 Hamburg 1- Maizenahaus (1966), Seite 10
NeosorbR for direct Compression (Roquette Frères), Herausgegeben vor 1981, Seiten 5 und 6
Prospekt über "Sorbit"/DHW Rodleben (1982)
Fachbereichstandard DDR, TGL 38206, Dezember 1981
Chem. Ing. Tech., 52 (1980), Nr. 7, Seite 584

Die Akte enthält technische Angaben, die nach dem Eingang der Anmeldung eingereicht wurden und die nicht in dieser Patentschrift enthalten sind.

## Beschreibung

Die Erfindung betrifft einen modifizierten Sorbit mit verbesserten Tablettiereigenschaften.

Sorbit ist eine in großem Umfang verwendete Tablettengrundlage, die unter anderem für Kau- und Lutschtabletten benutzt wird. Der besondere Vorteil von Sorbit liegt darin, daß er im Prinzip auch zum direkten Verpressen ohne weitere Hilfs- und Zusatzstoffe geeignet ist. Gewonnen wird Sorbit in der Regel durch Hydrierung von Stärkehydrolysaten oder durch Hydrierung von Invertzucker mit anschließender Abtrennung von Mannit. In fester Form kann er sowohl durch Kristallisation als auch durch Sprühtrocknung erhalten werden.

Zur Herstellung zufriedenstellender Komprimate ist solcher Sorbit in der Regel wenig geeignet. Es sind daher spezielle Verfahren entwickelt worden, um einen zur besseren Verpressung geeigneten Sorbit herzustellen. So wird in der DE-AI 3 009 875 ein sehr spezielles kompliziertes Kristallisierverfahren beschrieben, nach dem ein zum Verpressen geeigneter Sorbit hergestellt werden kann. Ein anderes spezielles Kristallisierverfahren ist in der EP-AI 32288 beschrieben.

Diese Verfahren erfordern jedoch einen erheblichen technischen Aufwand, da die Kristallisationsbedingungen sehr exakt eingehalten werden müssen, um zu einem verwertbaren Produkt zu gelangen. Darüberhinaus sind die aus den so hergestellten Sorbittypen gepressten Erzeugnisse noch nicht völlig zufriedenstellend in Bezug auf Biegefestigkeit und Abriebfestigkeit.

Es bestand deshalb die Aufgabe, einen Sorbit zu finden, der problemloser herstellbar ist und der insbesondere in den Tablettiereigenschaften wie Biege- und Abriebfestigkeit gegenüber den bekannten Sorbittypen verbessert ist.

Es wurde nun gefunden, daß überraschenderweise auch durch Sprühtrocknung gewonnener Sorbit zur direkten Verpressung geeignet ist und dabei sogar bessere Resultate liefert als der bisher gebräuchliche durch Kristallisation gewonnene Sorbit. Voraussetzung dafür ist, daß dieser Sorbit bestimmte Spezifikationen erfüllt, bzw. beim Verfahren zur Herstellung bestimmte Parameter eingehalten werden.

Gegenstand der Erfindung ist daher ein modifizierter Sorbit mit verbesserten Tablettiereigenschaften, der gekennzeichnet ist durch

a) einen Schmelzpunkt von 96 °C

b) eine Schüttdichte (nach DIN 53 912) von 0,3–0,6 g/ml

c) einen $\gamma$-Sorbitgehalt von mindestens 90%

d) eine Reinheit von mindestens 98%

e) eine spezifische Oberfläche (nach BET) von 0,7 bis 1,5 m²/g

f) eine Biegefestigkeit von mehr als 7 N/mm² bei einer Preßkraft oberhalb 10 000 N

g) eine Friabilität von weniger als 1% bei einer Preßkraft ab 10 000 N, wobei die Werte unter (f) und (g) Messungen bei Tabletten des Sorbits von 10 mm Durchmesser und 0,3 g Gewicht entsprechen.

Gegenstand der Erfindung ist auch ein Verfahren zur Herstellung von Sorbit mit verbesserten Tablettiereigenschaften wobei durch Hydrierung von glucosehaltigen Lösungen eine Sorbitlösung gewonnen wird und daraus durch Sprühtrocknung Sorbit erhalten wird, das dadurch gekennzeichnet ist, daß eine Lösung von kristallisierter Glucose bei einer Temperatur unterhalb 170 °C hydriert wird und die so erhaltene Sorbitlösung bei einer Temperatur von 140 bis 170 °C so sprühgetrocknet wird, daß das erhaltene Produkt einen Wassergehalt von weniger als 1% aufweist.

Schließlich ist Gegenstand der Erfindung auch die Verwendung des Sorbits zur Herstellung von verpressten Zubereitungen.

Es ist bekannt, durch Sprühtrocknung gewonnenen sogenannten Instant-Sorbit auch als Zusatzstoff zu festen, auch verpressten, Zubereitungen zu verwenden. Dazu konnte der bisher bekannte Instant-Sorbit jedoch nicht allein verwendet werden, sondern mußte mit kristallisiertem Sorbit, z.B. nach der DE-AI 3 009 875 oder dem EP-AI 32288, verschnitten werden. Die alleinige Verwendung von bisher bekanntem Instant-Sorbit führte nach kurzer Zeit zum Kleben auf der Tablettenpresse und damit zu ihrer Blockierung.

Es wurde jetzt jedoch gefunden, daß die Herstellung von Instant-Sorbit so gesteuert werden kann, daß ein sehr gut verpressbares Produkt erhalten wird. Eine wesentliche Voraussetzung dafür ist, daß der Sorbit weitgehend frei ist von Nebenprodukten, wie sie z.B. durch die Wahl des Ausgangsmaterials für die Hydrierung bzw. durch die Hydrierbedingungen selbst gebildet werden können. Schon geringe Mengen der isomeren Zuckeralkohole Idit, Galaktit oder Talit verhindern eine einwandfreie Verpressung und sollten daher vorzugsweise maximal in einer Menge von etwa 0,1% enthalten sein.

Wesentliche Voraussetzung für die Herstellung des erfindungsgemäßen Sorbits ist daher die Verwendung einer kristallisierten Glucose als Ausgangsmaterial für die Hydrierung. Ebenso wichtig ist, daß die Hydrierung unter schonenden Bedingungen stattfindet, d.h. bei Temperaturen, die unterhalb etwa 170 °C, vorzugsweise unterhalb etwa 160 °C, liegen.

Die Hydrierung kann ansonsten unter den üblichen Bedingungen durchgeführt werden.

Dabei werden 50 bis 70%ige Glucose-Lösungen in Gegenwart eines Hydrierkatalysators, wie z.B. eines Nickel-Trägerkatalysators, insbesondere Raney-Nickel, bei Wasserstoffdrucken von etwa 150 bis etwa 200 bar, insbesondere etwa 150 bis etwa 180 bar, hydriert. Danach wird die Lösung abgekühlt und vom Katalysator abgetrennt. Der Katalysator kann ggf. nach Aufarbeitung wieder verwendet werden. Die Analyse der Lösungen zeigt, daß bereits bei Temperaturen von 170 °C erhebliche Mengen an Nebenprodukten gebildet werden. Obwohl diese Nebenprodukte die Verwendbarkeit des Sorbits als Zuckeraustauschstoff nicht stören, hat sich doch überraschenderweise herausgestellt, daß diese Nebenprodukte die Verpreßbarkeit des Sorbits stark beeinträchtigen. Bei Hydriertemperaturen unterhalb etwa 170 °C und insbesondere unterhalb etwa 160 °C dagegen ist die Bildung von Nebenprodukten so stark vermindert, daß der Sorbit aus den so erhaltenen Sorbitlösungen nicht nach komplizierten und zeitaufwendigen Verfahren kristallisiert werden muß, sondern durch Sprühtrocknung gewonnen werden kann.

Zur Sprühtrocknung kann unmittelbar die aus der Hydrierlösung nach Abtrennen des Katalysators und Entsalzung erhaltene Sorbitlösung verwendet werden. Der Feststoffgehalt wird zuvor auf etwa 65 bis etwa 75 Gew.% eingestellt. Die Versprühung wird durch Zerstäuben mittels eines Zentrifugalzerstäubers in einen auf eine Temperatur von 140–170 °C erwärmten trockenen zentrifugal eingeblasenen Luftstrom durchgeführt. Die Menge der zugeführten Sorbitlösung und der eingeblasenen Heißluft wird so abgestimmt, daß der Sorbit bis auf einen Wassergehalt von etwa 0,3 bis etwa 1 Gew.% getrocknet wird. Auf jeden Fall sollte der Wassergehalt unterhalb 1 Gew.% liegen.

Die Sorbitteilchen, die dabei durch Entwässerung der Sorbitlösungströpfchen erhalten werden, werden bei der Sprühtrocknung auf eine Temperatur von etwa 50 bis etwa 70 °C erwärmt, während die eingeblasene Luft auf etwa die gleiche Temperatur abgekühlt wird. Der Sorbit wird in Behältern gesammelt und ist nach dem Abkühlen direkt zur Herstellung von Komprimaten geeignet. Im Gegensatz zu dem vorbekannten Sorbit-Instant ist das nach dem erfindungsgemäßen Verfahren erhaltene Material überraschenderweise unter Zusatz von geringen Mengen eines üblichen Gleitmittels wie z.B. Magnesiumstearat verpreßbar, ohne daß es zum Kleben an den Tablettierstempeln kommt.

Bei dem erfindungsgemäßen Sorbit handelt es sich um einen Sorbit, der nach dem Debye-Scherrer-Diagramm im wesentlichen aus γ-Sorbit besteht. Im Gegensatz zu den aus der DE-Al 3 009 875 und der EP-Al 32288 bekannten γ-Sorbiten besitzt der erfindungsgemäße Sorbit eine unregelmäßige Oberfläche. Im Schmelzverhalten (Differential Scanning Calorimetry) zeigt der erfindungsgemäße Sorbit einen bei etwa 96 °C liegenden scharfen Peak. Die Schüttdichte (nach DIN 53 912) beträgt etwa 0,3 bis 0,6 g/ml, die Stampfdichte (nach DIN 53 194) etwa 0,4 bis 0,7 g/ml. Aufgrund der unregelmäßigen Oberfläche besitzt der erfindungsgemäße Sorbit eine relativ hohe spezifische Oberfläche von etwa 0,7 bis etwa 1,5 m²/g. Die Teilchengröße kann durch das Sprühtrockenverfahren in weiten Grenzen gesteuert werden, liegt aber in der Regel wesentlich niedriger als bei den vorbekannten verpreßbaren Sorbittypen.

Der so charakterisierte Sorbit besitzt eine Reihe von vorteilhaften Tablettiereigenschaften.

Überraschenderweise kann festgestellt werden, daß mit dem erfindungsgemäßen Sorbit bei gleicher Preßkraft sehr viel härtere Tabletten hergestellt werden können, als mit den bekannten verpreßbaren Sorbittypen. Da die optimale Festigkeit von Lutschtabletten durch das Lutschverhalten vorgegeben ist, bedeutet dies, daß optimal harte Tabletten bereits mit sehr niedrigen Preßkräften hergestellt werden können. Die Biegefestigkeit als Maßstab für die Härte erreicht beim erfindungsgemäßen Sorbit bei Tabletten von 10 mm Durchmesser und 0,3 g Gewicht bereits bei einer Preßkraft ab etwa 10 000 N einen Wert von etwa 7 N/mm² und steigt weiter an auf einen Wert von etwa 11 N/mm² bei einer Preßkraft von oberhalb 20 000 N. Ein durch Kristallisation gewonnener verpreßbarer Sorbit hat dagegen bei einer Preßkraft von 10 000 N nur eine Biegefestigkeit von etwa 2,5 N/mm², die durch Erhöhung der Preßkraft auf 20 000 N lediglich auf einen Wert von etwa 5 N/mm² gesteigert werden kann. Tablettiermaschinen, mit denen der erfindungsgemäße Sorbit verpreßt wird, können also bei relativ niedrigen Preßkräften arbeiten und unterliegen auf diese Weise einem sehr viel geringeren Verschleiß.

Neben der Biegefestigkeit ist insbesondere auch die Abriebfestigkeit entscheidend für die Qualität der Tabletten. Mit dem erfindungsgemäßen Sorbit gepreßte Tabletten besitzen bereits bei einer Preßkraft von 10 000 N eine Abriebfestigkeit von weniger als 1%, die bereits bei einer Preßkraft ab 15 000 N auf einen extrem geringen Wert von etwa 0,2 bis 0,3% zurückgeht. Tabletten aus dem bekannten verpreßbaren Sorbit dagegen haben bei 5000 N Preßkraft einen Abrieb von mehr als 6%, der erst oberhalb 15 000 N auf einen Endwert von etwa 1% zurückgeht.

Wie bereits oben erwähnt, ist die Festigkeit der aus dem erfindungsgemäßen Sorbit gepreßten Tabletten bei gleicher Preßkraft sehr viel höher als die aus dem bekannten Sorbit gepreßten Tabletten. Tabletten gleicher Festigkeit können daher mit dem erfindungsgemäßen Sorbit bei geringerer Preßkraft hergestellt werden und besitzen demgemäß ein deutlich höheres Volumen als die Vergleichstabletten. Da bei gleicher Tablettenfestigkeit die Lutschdauer dem Volumen der Tablette entspricht, können mit dem erfindungsgemäßen Sorbit Tabletten hergestellt werden, die bei gleicher Lutschdauer ein wesentlich geringeres Gewicht aufweisen. Auf diese Weise können erhebliche Materialeinsparungen erzielt werden.

Durch die unregelmäßige Oberfläche ist der erfindungsgemäße Sorbit in der Lage, auch größere Mengen von Zusatzstoffen, wie z.B. von Kakaopulver, Farbstoffen oder anderen Zusätzen zu binden. Die Aufnahmekapazität liegt dabei wesentlich über dem der bekannten verpreßbaren Sorbittypen. Auch bei starker Beladung mit Zusatzstoffen erhält man homogene Mischungen und die daraus hergestellten Komprimate besitzen ein gleichmäßiges Aussehen.

Aufgrund der besonderen Herstellungsart durch Versprühen einer wässerigen Lösung ist es möglich, wasserlösliche Zusätze, wie z.B. Farbstoffe, Vitamine und dergleichen, völlig homogen in Sorbit bzw. den daraus hergestellten Komprimaten zu verteilen.

Im Gegensatz zu Tabletten, die durch Zumischen von Farbstoff zu kristallisiertem Sorbit vor der Tablettierung erhalten wurden, und die eine gesprenkelte Oberfläche zeigen, besitzen erfindungsgemäß hergestellte Tabletten eine vollkommen gleichmäßig gefärbte Oberfläche.

Wie bereits erwähnt, kann der erfindungsgemäße Sorbit lediglich unter Zusatz von geringen Mengen eines üblichen Gleitmittels, wie z.B. 0,3 bis 2% Magnesiumstearat, direkt verpreßt werden. Wesentlich dafür ist lediglich, daß der Wassergehalt, der aufgrund des erfindungsgemäßen Herstellungsverfahrens bei etwa 0,3 bis etwa 1 Gew.% liegt, nicht durch zu langes Lagern bei hoher Luftfeuchtigkeit auf Werte über etwa 1% ansteigt. Sorbittypen mit höherem Wassergehalt können zwar in Räumen mit relativ niedriger Luftfeuchtigkeit noch einwandfrei verpreßt werden, neigen jedoch beim Verpressen in Räumen mit hoher Luftfeuchtigkeit zum Ankleben an den Preßstempeln.

Diese gute Verpreßbarkeit wird auch nicht beeinträchtigt, wenn Zusatzstoffe wie z.B. Farbstoffe, Geschmacksstoffe oder pharmazeutische Wirkstoffe zugemischt werden, durch die Aussehen, Geschmack und Wirkungsspektrum der Zubereitung beeinflußt werden können.

Wie bereits oben erwähnt, sollte der Reinheitsgrad des erfindungsgemäßen Sorbits oberhalb von etwa 98% liegen, da festgestellt wurde, daß durch Verunreinigungen die Tablettiereigenschaften sehr negativ beeinflußt werden können.

Andererseits wurde jedoch festgestellt, daß durch bestimmte Zumischungen, die ohnehin schon sehr gute Tablettenfestigkeit sogar noch erhöht werden kann. So wird überraschenderweise durch Zumischen von Mannit in einer Menge von etwa 10 bis 15 Gew.% vor oder nach dem Sprühtrocknen ein Produkt erhalten, das bereits bei einer Preßkraft von oberhalb 15 000 N eine Biegefestigkeit von über 15 N/mm² erreicht. Ein besonders vorteilhafter Aspekt der vorliegenden Erfindung sind daher auch Sorbitzubereitungen, die bis zu 15 Gew.% Mannit enthalten.

Der erfindungsgemäße Sorbit kann allein oder mit Zusätzen zu allen üblichen Zwecken eingesetzt werden, insbesondere zur Herstellung von Kau- und Lutschtabletten. Durch die verbesserten Tablettiereigenschaften wird durch die Erfindung ein erheblicher Fortschritt auf diesem Gebiet erzielt.

Herstellungsbeispiele:
Beispiel 1:

Eine Lösung von 750 g kristallisierter Dextrose in 500 ml vollentsalztem Wasser wird in einem 2-l-Hochdruckautoklaven mit 50 g feuchtem Raney-Nickel versetzt und bei 130°C und 150 bar bis zur Beendigung der Wasserstoffaufnahme hydriert. Nach Abkühlen der Suspension wird der Katalysator abgesaugt und mit Wasser gewaschen.

Der Feststoffgehalt der Lösung besteht zu 99,2 Gew.% aus Sorbit. Nebenprodukte sind nur in unbedeutenden Spuren enthalten. Die so hergestellte etwa 60 Gew.%ige Lösung wird mittels eines Zentrifugalzerstäubers versprüht und mit einem Luftstrom von 150°C getrocknet. Man erhält einen im wesentlichen aus der γ-Form bestehenden Sorbit mit 0,8 Gew.% Restfeuchtigkeit, einem Schmelzpunkt von 96°C, einer Schüttdichte von 0,4 g/ml, einer spezifischen Oberfläche (nach BET) von 1,0 m²/g, einer mittleren Korngröße (α'-Wert der RRSB-Verteilung) von 500 μm, wobei 90% der Teilchen im Bereich von 280 bis 620 μm liegen. Beim Pressen des Sorbits unter Zusatz von 0,3 Gew.% Magnesiumstearat zu Tabletten mit 300 mg Gewicht und 10 mm Durchmesser auf einer Exzenterpresse werden folgende Ergebnisse erzielt:

| Preßkraft [N] | 10 000 | 20 000 | 30 000 |
|---|---|---|---|
| Tablettenhöhe [mm] | 3,02 | 2,77 | 2,74 |
| Biegefestigkeit (N/mm²] | 8,5 | 12,5 | 12,5 |
| Friabilität [%] | 0,4 | 0,2 | 0,2 |

Die Biegefestigkeit wird mit einem umgebauten Härtetester der Firma Erweka, Heusenstamm, nach der in P.H. List: Arzneiformenlehre, 3. Auflage, S. 105, Wiss. Verlagsgesellschaft mbH, Stuttgart, 1982, beschriebenen Methode bestimmt, wobei die Stützweite 6,7 mm beträgt.

Die Friabilität wird in einem Friabilator der Firma Erweka, Heusenstamm, bei einer Laufzeit von 6 Min. bestimmt.

Beispiel 2

Es wird entsprechend Beispiel 1 hydriert, jedoch bei einer Temperatur von 150°C. Der Feststoffgehalt der erhaltenen Lösung besteht zu 98,7 Gew.% aus Sorbit. Auch hier sind Nebenprodukte nur in unbedeutender Menge vorhanden. Das durch Sprühtrocknung analog Beispiel 1 erhaltene Produkt läßt sich problemlos verpressen, wobei Ergebnisse analog den in Beispiel 1 angegebenen erzielt werden.

Beispiel 3 (Vergleichsbeispiel):

Es wird analog Beispiel 1, jedoch bei einer Temperatur von 170°C hydriert. Der Feststoffgehalt der erhaltenen Lösung besteht nur zu 95,9 Gew.% aus Sorbit. Die enthaltenen Nebenprodukte machen das nach Sprühtrocknung analog Beispiel 1 gewonnene Produkt untauglich für die direkte Verpressung, obwohl das Material ohne weiteres als Zuckeraustauschstoff eingesetzt werden könnte.

Beispiel 4 (Vergleichsbeispiel):

Eine Lösung von 750 kg kristallisierter Dextrose in 500 l Wasser wird mit 35 kg Raney-Nickel versetzt und bei 180°C und einem Druck von 150 bar hydriert. Die nach Beendigung der Wasserstoffaufnahme abgekühlte und filtrierte Lösung weist einen Feststoffgehalt auf, der zu 94,1 Gew.% aus Sorbit besteht.

Die zahlreichen Nebenprodukte machen das durch Sprühtrocknung analog Beispiel 1 gewonnene Produkt untauglich für die direkte Verpressung, obwohl auch dieses Produkt als Zuckeraustauschstoff gut verwendbar wäre.

In den folgenden Anwendungsbeispielen kann ein nach Beispiel 1 oder Beispiel 2 hergestellter Sorbit eingesetzt werden.

Beispiel 5: Mundpflegetabletten
Sorbit-Instant                96,0 Gew.teile
Magnesiumperoxid               2,0 Gew.teile
Pfefferminzöl                  1,5 Gew.teile
Magnesiumstearat               0,5 Gew.teile

Die Bestandteile werden vermischt und bei einem Preßdruck von 6000 N zu Tabletten von 6 mm Durchmesser und 100 mg Gewicht verpreßt.

Beispiel 6: Mentholtabletten
Sorbit-Instant               247,0 Gew.teile
Menthol                        1,8 Gew.teile
Magnesiumstearat               1,2 Gew.teile

Die Bestandteile werden vermischt und bei einem Preßdruck von 14 000 N zu Tabletten von 9 mm Durchmesser und einem Gewicht von 250 mg verpreßt.

Beispiel 7: Tabletten zur Kariesprophylaxe
Sorbit-Instant                98,34 Gew.teile
Natriumfluorid                 1,16 Gew.teile
Magnesiumstearat               0,5 Gew.teile

Die Bestandteile werden vermischt und bei einem Preßdruck von 8000 N zu Tabletten von 7 mm Durchmesser und 100 mg Gewicht verpreßt.

Beispiel 8: Sorbitlutschtabletten
Sorbit-Instant               491,0 Gew.teile
Zitronensäure                  5,0 Gew.teile
Früchtetrockenaroma
(verschiedene
Geschmacksrichtungen)          1,5 Gew.teile
Magnesiumstearat               2,5 Gew.teile

Die Bestandteile werden vermischt und bei einem Preßdruck von 30 000 N zu Tabletten von 13 mm Durchmesser und 500 mg Gewicht verpreßt.

Beispiel 9: Vitamin-C-Tabletten
Sorbit-Instant               447,0 Gew.teile
Ascorbinsäure                 50,0 Gew.teile
Zitronentrockenaroma           0,5 Gew.teile
Magnesiumstearat               2,5 Gew.teile

Die Bestandteile werden vermischt und bei einem Preßdruck von 30 000 N zu Tabletten von 13 mm Durchmesser und 500 mg Gewicht verpreßt.

Beispiel 10: Kaffeetabletten
Sorbit-Instant               462,5 Gew.teile
Kaffeeextraktpulver           25,0 Gew.teile
Koffein                       10,0 Gew.teile
Magnesiumstearat               2,5 Gew.teile

Die Bestandteile werden vermischt und mit einem Preßdruck von 30 000 N zu Tabletten von 13 mm Durchmesser und einem Gewicht von 500 mg verpreßt.

Beispiel 11: Kakaotabletten
Sorbit-Instant               449 Gew.teile
Kakaopulver                   50 Gew.teile
Zitronensäure                  1 Gew.teil

Die Bestandteile werden vermischt und mit einem Preßdruck von 30 000 N zu Tabletten mit einem Durchmesser von 13 mm und einem Gewicht von 500 mg verpreßt.

In sämtlichen angegebenen Rezepturen kann auch mit 0,1% Saccharin oder 0,2 Aspartam aufgesüßter Sorbit-Instant oder auch eingefärbter Sorbit-Instant eingesetzt werden.

**Patentansprüche**

1. Modifizierter Sorbit mit verbesserten Tablettiereigenschaften, gekennzeichnet durch
   a) einen Schmelzpunkt von etwa 96 °C,
   b) eine Schüttdichte (nach DIN 53912) von 0,3 bis 0,6 g/ml,
   c) einen γ-Sorbitgehalt von mindestens 90%,
   d) eine Reinheit von mindestens 98%,
   e) eine spezifische Oberfläche (nach BET) von 0,7 bis 1,5 m²/g,
   f) eine Biegefestigkeit von mehr als 7 N/mm² bei einer Preßkraft oberhalb 10 000 N,
   g) eine Friabilität von weniger als 1% bei einer Preßkraft ab 10 000 N, wobei die Werte unter (f) und (g) Messungen bei Tabletten des Sorbits von 10 mm Durchmesser und 0,3 g Gewicht entsprechen.

2. Verfahren zur Herstellung von Sorbit mit verbesserten Tablettiereigenschaften, wobei durch Hydrierung von glucosehaltigen Lösungen eine Sorbitlösung gewonnen wird und daraus durch Sprühtrocknung Sorbit erhalten wird, dadurch gekennzeichnet, daß eine Lösung von kristallisierter Glucose bei einer Temperatur unterhalb 170 °C hydriert und die so erhaltene Sorbitlösung bei einer Temperatur von 140 bis 170 °C so sprühgetrocknet wird, daß das erhaltene Produkt einen Wassergehalt von weniger als 1% aufweist.

3. Verwendung des Sorbits nach Anspruch 1 zur Herstellung von verpreßten Zubereitungen.

**Claims**

1. Modified sorbitol having improved tabletting properties, characterised by
   a) a melting point of about 96 °C,
   b) a bulk density (by the method of DIN 53,912) of 0.3 to 0.6 g/ml,
   c) a γ-sorbitol content of at least 90%,
   d) a purity of at least 98%,
   e) a specific surface area (by the BET method) of 0,7 to 1.5 m²/g,
   f) a flexural strength greater than 7 N/mm² at a compressive force above 10,000 N, and
   g) a friability of less than 1% at a compressive force at and above 10,000 N, where the values under (f) and (g) correspond to measurements on sorbitol tablets of diameter 10 mm and weight 0.3 g.

2. Process for the preparation of sorbitol having improved tabletting properties, entailing a sorbitol solution being obtained by hydrogenation of solutions containing glucose, and sorbitol being obtained from this by spray-drying, characterised in that a solution of crystallized glucose is hydrogenated at a temperature below 170°C, and the sorbitol solution thus obtained is spray-dried at a temperature of 140 to 170°C in such a manner that the product obtained has a water content of less than 1%.

3. Use of the sorbitol according to Claim 1 for the production of compressed formulations.

**Revendications**

1. Sorbitol modifié ayant des propriétés améliorées de compressibilité, caractérisé par
   a) un point de fusion d'environ 96°C,
   b) une densité apparente (selon DIN 53 912) de 0,3 à 0,6 g/ml,
   c) une teneur en gamma-sorbitol d'au moins 90%,
   d) une pureté d'au moins 98%,
   e) une surface spécifique (selon BET) de 0,7 à 1,5 m$^2$/g,

f) une résistance à la flexion supérieure à 7 N/mm$^2$ sous une force de compression supérieure à 10 000 N,
   g) une friabilité inférieure à 1% sous une force de compression de 10 000 N ou au-dessus, les valeurs données ci-dessus sous f) et g) étant mesurées sur des comprimés de sorbitol de 10 mm de diamètre pesant 0,3 g.

2. Procédé de préparation d'un sorbitol possédant des propriétés améliorées de mise sous forme de comprimé, dans lequel, par hydrogénation de solutions contenant du glucose, on obtient une solution de sorbitol, à partir de laquelle on obtient du sorbitol par séchage-atomisation, caractérisé en ce que l'on hydrogène une solution de glucose cristallisé à une température inférieure à 170°C et on soumet la solution de sorbitol ainsi obtenue à séchage par atomisation à une température de 140 à 170°C dans des conditions telles que le produit obtenu présente une teneur en humidité inférieure à 1%.

3. Utilisation du sorbitol selon la revendication 1 pour la préparation de compositions comprimées.